(19) **Europäisches Patentamt / European Patent Office / Office européen des brevets**

(11) **EP 4 614 515 A1**

(12) **EUROPEAN PATENT APPLICATION**
published in accordance with Art. 153(4) EPC

(43) Date of publication:
**10.09.2025  Bulletin 2025/37**

(21) Application number: **23884121.7**

(22) Date of filing: **09.05.2023**

(51) International Patent Classification (IPC):
*G16H 50/20* (2018.01)     *G16B 20/50* (2019.01)
*G16B 20/30* (2019.01)     *C12Q 1/68* (2018.01)

(52) Cooperative Patent Classification (CPC):
**C12Q 1/68; C12Q 1/6869; C12Q 1/6886; G06F 18/2433; G16B 20/20; G16B 20/30; G16B 20/50; G16H 50/20**

(86) International application number:
**PCT/CN2023/093013**

(87) International publication number:
**WO 2024/093179 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority:  **02.11.2022  CN 202211361261**

(71) Applicant: **Shenzhen Bay Laboratory**
**Shenzhen, Guangdong 518132 (CN)**

(72) Inventors:
• **SUN, Kun**
  **Shenzhen, Guangdong 518132 (CN)**
• **JU, Jia**
  **Shenzhen, Guangdong 518132 (CN)**

(74) Representative: **Huang, Liwei**
**Cäcilienstraße 12**
**40597 Düsseldorf (DE)**

(54) **PAN-TUMOR BLOOD DIAGNOSIS MARKER AND USE THEREOF**

(57)     The present application discloses a Pan-cancer blood diagnosis marker and use thereof. A first aspect of the present application provides a classification method of a sample. The classification method comprises the following steps: acquiring sequencing data of cell-free DNA from the sample; aligning the sequencing data of the cell-free DNA with the positions of nucleosomes on a reference genome to obtain a position result of an end of the cell-free DNA relative to nucleosome distribution: and determining the type of the sample according to the position result. Upon examination, this method is applicable to various types of cancer, which is capable of sensitively distinguishing cancer patient samples and non-cancer patient samples, and has relatively low requirements on sequencing depth.

$$N - index = \frac{6}{12}$$

FIG. 2

**Description**

**TECHNICAL FIELD**

The present disclosure relates to the technical field of cancer diagnosis, in particular to a Pan-cancer blood diagnosis marker and use thereof.

**BACKGROUND**

Circulating cell-free DNA (cfDNA) is a DNA fragment generated by natural degradation after cell death. Studies show that in healthy people, cfDNA is mainly derived from blood cells and liver; and in cancer patients, dead tumor cells also release cfDNA to enter peripheral blood. There are significant differences between cfDNA fragments from tumors and cfDNA fragments from non-tumor cells, such as gene mutation, chromosome copy number variation and methylation level abnormity. Therefore, cancer diagnosis is conducted by detecting cfDNA signals from tumors in peripheral blood. i.e.. a liquid biopsy technique. Since the collection of peripheral blood is almost non-invasive to a human body and the peripheral blood is sampled repeatedly, a cancer diagnosis technique based on peripheral blood cfDNA has an extremely high application value. Related studies have identified a large number of tumor cfDNA markers, and developed many cancer diagnosis techniques such as a high frequency gene mutation kit and a tumor-specific methylation marker kit.

In recent years, researchers have discovered that cfDNA is not randomly fragmented during the generation, and found some tumor markers related to cfDNA fragmentation patterns, so as to develop multiple cancer diagnosis techniques. For example, loci of preference of a specific cancer patient are identified according to the position distribution of fragment ends of cfDNA on a genome, cancer patients are distinguished from a control group through these preference loci. However, these preference loci need to be identified by using complex statistical methods. Furthermore, there are currently no evidences to suggest the existence of Pan-cancer tumor cfDNA preference loci, and therefore it is needed for each type of cancer to identify a corresponding tumor cfDNA preference locus, which leads to expensive cost and poor universality. In addition, this technique has relatively high requirements on sequencing depth. In cfDNA fragment data obtained by sequencing, only a few of them overlap these preference loci, and most of them are considered as useless information to be discarded because they do not cover these loci.

Therefore, it is necessary to provide a Pan-cancer diagnosis marker which has low requirements on sequencing depth while achieving high flexibility and low cost.

**SUMMARY**

The present disclosure aims to solve at least one of the technical problems in the prior art. To this end, the present disclosure provides a Pan-cancer blood diagnosis marker. By utilizing the marker, detection is completed in low cost and high flexibility, and requirements on sequencing depth are relatively low during the sequencing.

A first aspect of the present disclosure provides a classification method of a sample, the classification method including the following steps:

acquiring sequencing data of cell-free DNA of a sample:

aligning the sequencing data of cell-free DNA with the positions of nucleosomes on a reference genome to obtain a position result how the ends of the cell-free DNAs are distributed relative to nucleosomes: and

determining the type of the sample according to the position result.

According to the classification method of the embodiments of the present disclosure, the present disclosure at least has the following beneficial effects:

Cell-free DNA is generated by degradation of chromatins during cell apoptosis, the fragmentation pattern of cell-free DNA is affected by nucleosome distribution: under normal conditions (such as healthy control or other populations without cancers), the ends of cell-free DNA have a higher tendency to be located on linker DNA between nucleosomes, which is defined here as higher consistency being present between cell-free DNA end and nucleosome distribution patterns: but in cancer patients, there is significant difference between a tumor-nucleosome distribution pattern and a normal cell or hematopoietic cell distribution pattern, and therefore the consistency between released DNA and nucleosome distribution is relatively low, leading to significant difference in an overall cell-free DNA fragmentation pattern. In addition, relative to normal tissues, a higher proportion of ends of cell-free DNA released by tumor tissues are inside the nucleosome, thus further reducing the consistency between the ends of cell-free DNA and nucleosome distribution in normal tissues. Based on this principle, a relative position relationship between the ends of cell-free DNA and the nucleosome distribution is confirmed by quantifying the position distribution of ends of cell-free DNA in a test subject, so as to conduct diagnosis of cancers. Upon examination, this method is applicable in various types of cancers, which is capable of sensitively distinguishing samples from cancer patients and non-cancer patients, and has relatively low requirements on sequencing depth.

In some embodiments of the present disclosure, the position result how the ends of the cell-free DNAs are distributed relative to nucleosomes includes: the end of the cell-free DNA is located in the interior of nucleosome or is located on linker DNA.

In some embodiments of the present disclosure, determining the type of the sample according to the position result includes:

determining that the sample is a cancer sample, when ends of the cell-free DNA are located in the interior of nucleosome;

determining that the sample is a non-cancer sample, when ends of the cell-free DNA are located on linker DNA:

In some embodiments of the present disclosure, the position result how the ends of the cell-free DNAs are distributed relative to nucleosomes includes: a proportion of ends of a plurality of cell-free DNAs located in the interior of nucleosome, and/or a proportion of ends of the plurality of cell-free DNAs located on linker DNA.

In some embodiments of the present disclosure, determining the type of the sample according to the position result includes:

determining that the sample is a cancer sample when a proportion of the ends of the plurality of cell-free DNAs located in the interior of nucleosomes is greater than a first threshold, and/or, when a proportion of the ends of the plurality of cell-free DNAs located on linker DNA is smaller than a second threshold:

determining that the sample is a non-cancer sample when a proportion of the ends of the plurality of cell-free DNAs located in the interior of nucleosomes is smaller than or equal to the first threshold, and/or, when a proportion of the ends of the plurality of cell-free DNAs located on linker DNAs is greater than or equal to the second threshold: and determining that the sample is a non-cancer sample when a distance between the end of the plurality of the cell-free DNAs and the center position of the nucleosome is greater than a third threshold: and determining that the sample is a cancer sample when the distance between the ends of the plurality of the cell-free DNAs and the center position of the nucleosome is smaller than or equal to the third threshold.

In some embodiments of the present disclosure, the cancer sample is a sample of at least one type of cancer of liver cancer, lung cancer, pancreatic cancer, bile duct cancer, breast cancer and ovarian cancer.

In some embodiments of the present disclosure, the position result is *N-index,* which meets

$$N - index = \frac{N_{interior\ of\ nucleosome}}{N_{all}}$$

where $N_{interior\ of\ nucleosome}$ is the number of cell-free DNA whose ends are located in the interior of nucleosomes in a plurality of cell-free DNAs: $N_{all}$ is the total number of a plurality of cell-free DNAs:

or, the position result is *N'-index,* which meets

$$N' - index = \frac{N_{interior\ of\ nucleosome}}{N_{linker\ DNA}}$$

where $N_{linker\ DNA}$ is the number of cell-free DNAs whose ends are located on linker DNAs between nucleosomes:

or, the position result is *N"-index,* which meets

$$N'' - index = \sum_{i=1}^{n} a_i b_i$$

where n is the number of the ends of the plurality of the cell-free DNAs, $a_i$ is a distance between an i-th end and the center of the nucleosome, and b, is a weight of the i-th end.

In some embodiments of the present disclosure, determining the type of the sample according to the position result includes:

determining that the sample is a cancer sample when *N-index* is greater than a first cutoff value; and
determining that the sample is a non-cancer sample when *N-index* is smaller than or equal to the first cutoff value.

In some embodiments of the present disclosure, determining the type of the sample according to the position result includes:
determining that the sample is a cancer sample when *N'-index* is greater than a second cutoff value:
determining that the sample is a non-cancer sample when *N'-index* is smaller than or equal to the second cutoff value.
In some embodiments of the present disclosure, determining the type of the sample according to the position result includes:

determining that the sample is a non-cancer sample when *N"-index* is greater than a third cutoff value: and
determining that the sample is a cancer sample when N"-index is smaller than or equal to third cutoff value:
In some embodiments of the present disclosure, the end of cell-free DNA includes at least one selected from an upstream end and a downstream end.

In some embodiments of the present disclosure, the reference genome includes a human whole genome or a designated region of the human whole genome, or genomes of one or more human individuals (such as non-cancer patients).
In some embodiments of the present disclosure, a target genome region includes the human whole genome or the designated region of the human whole genome.
In some embodiments of the present disclosure, the target genome region is at least one selected from chr1, chr2, chr3, chr4, chr5, chr6, chr7, chr8, chr9, chr10, chr11, chr12, chr13, chr14, chr15, chr16, chr17, chr18, chr19, chr20, chr21, chr22, chrX and chrY in the human whole genome.
The reference genome and the target genome region can be the same or different, but obviously have overlapped parts.
In some embodiments of the present disclosure, the sample is a blood sample.
In some embodiments of the present disclosure, the cancer is a solid tumor.
In some embodiments of the present disclosure, the cancer is at least one selected from brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphatic cancer and skin cancer.
In some embodiments of the present disclosure, the position of the nucleosome on the reference genome is obtained by at least one method of a)-b):

a) performing sequencing analysis on cells from a non-cancer patient to determine the position of the nucleosome of the cell on the reference genome: and
b) analyzing the sequencing data of cell-free DNA to establish a map of the nucleosome on the reference genome, so as to determine the position of the nucleosome on the reference genome.

In some embodiments of the present disclosure, the cell from a non-cancer population is optionally selected from somatic cells.
In some embodiments of the present disclosure, the somatic cell is at least one selected from endothelial cells, epithelial cells, bone cells, chondrocytes, nerve cells, adipocytes, fibroblasts, mesenchymal cells, liver cells, pancreatic islet cells and blood cells.
In some embodiments of the present disclosure, the cell is the blood cell, and the blood cell is a leukocyte.
In some embodiments of the present disclosure, a method for sequencing analysis of the cells from the non-cancer population is at least one selected from Mnase-seq, ChIP-seq, ATAC-seq, NOME-seq, DNase-seq, FAIRE-seq and Cut & Tag.
In some embodiments of the present disclosure, the classification method further includes acquiring at least one selected from cell-free DNA copy number variation data, cell-free DNA methylation data, cell-free DNA length distribution data, cell-free DNA end base distribution data, cell-free DNA end distribution consistency data, marker data and image data of a sample for analysis, and then determining the classification of the sample in combination with the analysis result and the position result.
A second aspect of the present disclosure provides a computer-readable storage medium, storing computer-executable instructions, which when executed by a computer, cause the computer to implement the above-mentioned classification method according to any one of the aspects.
A third aspect of the present disclosure provides a device, including a processor and a memory, where the memory stores a computer program runnable on the processor which, when executed by the processor, causes the processor to implement the above-mentioned classification method.

A fourth aspect of the present disclosure provides a system for diagnosing a cancer, the system including:

an acquisition module for acquiring sequencing data of cell-free DNA from a non-cancer population; and
a comparison module for aligning the sequencing data of the cell-free DNA with a position of a nucleosome on a reference genome to obtain the position result of the end of the cell-free DNA relative to nucleosome distribution, and judging whether the sample is a cancer sample according to the position result.

In some embodiments of the present disclosure, the position result how the ends of the cell-free DNAs are distributed relative to nucleosomes includes: the end of the cell-free DNA is located in the interior of nucleosome or is located on linker DNA.

In some embodiments of the present disclosure, judging whether the sample is a cancer sample according to the position result includes:

determining that the sample is a cancer sample when the end of the cell-free DNA is located in the interior of nucleosome; and
determining that the sample is a non-cancer sample when the end of the cell-free DNA is located on liner DNA.

In some embodiments of the present disclosure, the position result how the ends of the cell-free DNAs are distributed relative to nucleosomes includes: a proportion of ends of a plurality of cell-free DNAs located in the interior of nucleosome, and/or a proportion of ends of the plurality of cell-free DNAs located on linker DNA.

In some embodiments of the present disclosure, judging whether the sample is a cancer sample according to the position result includes:

determining that the sample is the cancer sample when a proportion of the ends of a plurality of cell-free DNAs located in the interior of nucleosomes is greater than a first threshold, and/or, when a proportion of the ends of the plurality of cell-free DNAs located on linker DNA is smaller than a second threshold:
determining that the sample is a non-cancer sample when the proportion of the ends of the plurality of cell-free DNAs located in the interior of nucleosomes is smaller than or equal to the first threshold, and/or, when the proportion of the ends of the plurality of cell-free DNAs located on linker DNA is greater than or equal to the second threshold: and
determining that the sample is a non-cancer sample when a distance between the ends of the plurality of the cell-free DNAs and the center position of the nucleosome is greater than a third threshold; determining that the sample is a cancer sample when the distance between the ends of the plurality of the cell-free DNAs and the center position of the nucleosome is smaller than or equal to the third threshold.

In some embodiments of the present disclosure, the cancer sample is a sample of at least one type of cancer selected from liver cancer, lung cancer, pancreatic cancer, bile duct cancer, breast cancer and ovarian cancer.

In some embodiments of the present disclosure, the position result is *N-index,* which meets

$$N - index = \frac{N_{\text{interior of nucleosome}}}{N_{\text{all}}}$$

where $N_{\text{interior of nucleosome}}$ is the number of cell-free DNAs whose ends are located in the interior of nucleosomes in a plurality of cell-free DNAs; $N_{\text{all}}$ is the total number of the plurality of cell-free DNAs;
or, the position result is *N'-index,* which meets

$$N' - index = \frac{N_{\text{interior of nucleosome}}}{N_{\text{linker DNA}}}$$

where $N_{\text{linker DNA}}$ is the number of cell-free DNAs whose ends are located on linker DNAs between the nucleosomes:

or, the position result is *N''-index,* which meets

$$N'' - index = \sum_{i=1}^{n} a_i b_i$$

where n is the number of ends of a plurality of cell-free DNAs, $a_i$ is a distance between an i-th end and the center of the nucleosome, and $b_i$ is a weight of the i-th end.

In some embodiments of the present disclosure, judging whether the sample is a cancer sample according to the position result includes:

determining that the sample is the cancer sample when *N-index* is greater than a first cutoff value: and

determining that the sample is a non-cancer sample when *N-index* is smaller than or equal to the first cutoff value.

In some embodiments of the present disclosure, judging whether the sample is a cancer sample according to the position result includes:
determining that the sample is a non-cancer sample when *N'-index* is greater than a second cutoff value:
determining that the sample is a cancer sample when *N'-index* is smaller than or equal to the second cutoff value.
In some embodiments of the present disclosure, judging whether the sample is a cancer sample according to the position result includes:

determining that the sample is a non-cancer sample when *N''-index* is greater than a third cutoff value: and
determining that the sample is a cancer sample when N''-index is smaller than or equal to third cutoff value:
In some embodiments of the present disclosure, the end of cell-free DNA includes at least one selected from an upstream end and a downstream end.

In some embodiments of the present disclosure, the reference genome includes the human whole genome or the designated region of the human whole genome, or genomes of one or more human individuals.
In some embodiments of the present disclosure, a target genome region includes the human whole genome or the designated region of the human whole genome.
In some embodiments of the present disclosure, the target genome region includes at least one selected from chr1, chr2, chr3, chr4, chr5, chr6, chr7, chr8, chr9, chr10, chr11, chr12, chr13, chr14, chr15, chr16, chr17, chr18, chr19, chr20. chr21, chr22. chrX and chrY in the human whole genome.
The reference genome and the target genome region can be the same or different, but obviously have overlapped parts.
In some embodiments of the present disclosure, the sample is a blood sample.
In some embodiments of the present disclosure, the cancer is a solid tumor.
In some embodiments of the present disclosure, the cancer is at least one selected from brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphatic cancer and skin cancer.
In some embodiments of the present disclosure, the position of the nucleosome on the reference genome is obtained by at least one method of a)-b):

a) performing sequencing analysis on cells from a non-cancer patient to determine the position of the nucleosome on the reference genome: and
b) analyzing the sequencing data of the cell-free DNA to establish a map of the nucleosome on the reference genome so as to determine the position of the nucleosome on the reference genome.

In some embodiments of the present disclosure, the cell from the non-cancer population is optionally selected from somatic cells.
In some embodiments of the present disclosure, the somatic cell is at least one selected from endothelial cells, epithelial cells, bone cells, chondrocytes, nerve cells, adipocytes, fibroblasts, mesenchymal cells, liver cells, pancreatic islet cells and blood cells.
In some embodiments of the present disclosure, the cell is the blood cell, and the blood cell is a leukocyte.
In some embodiments of the present disclosure, a method for sequencing analysis of the cells from the non-cancer population is at least one selected from Mnase-seq, ChIP-seq, ATAC-seq, NOME-seq, DNase-seq, FAIRE-seq and Cut & Tag.
In some embodiments of the present disclosure, the classification method further includes acquiring at least one selected from cell-free DNA copy number variation data, cell-free DNA methylation data, cell-free DNA length distribution data, cell-free DNA end base distribution data, cell-free DNA end distribution consistency data, marker data and image data of a sample for analysis, and then determining the type of the sample in combination with the analysis result and the position result.

A fifth aspect of the present disclosure provides a Pan-cancer blood diagnosis marker, the Pan-cancer blood diagnosis marker including at least one selected from *N-index*, *N'-index* and *N''-index* which characterize the consistency between a fragmentation pattern of cell-free DNA and nucleosome distribution of blood cells, which meets

$$N - index = \frac{N_{\text{interior of nucleosome}}}{N_{\text{all}}}$$

where $N_{\text{interior of nucleosome}}$ is the number of cell-free DNAs whose ends are located in the interior of nucleosomes in a plurality of cell-free DNAs: and $N_{\text{all}}$ is the total number of a plurality of cell-free DNAs:

$$N' - index = \frac{N_{\text{interior of nucleosome}}}{N_{\text{linker DNA}}}$$

where $N_{\text{linker DNA}}$ is the number of cell-free DNAs whose ends are located on linker DNAs between the nucleosomes:

$$N'' - index = \sum_{i=1}^{n} a_i b_i$$

where n is the number of the ends of a plurality of cell-free DNAs, $a_i$ is a distance between an i-th end and the center of the nucleosome, and b, is a weight of the i-th end.

A sixth aspect of the present disclosure provides use of the above-mentioned Pan-cancer blood diagnosis marker, or, a product for detecting the Pan-cancer blood diagnosis marker in preparation of a cancer diagnosis product.

The classification method of the present disclosure is used for multiple cancer types, with high universality and wide application scenarios. Meanwhile, the Pan-cancer blood diagnosis marker of the present disclosure has relatively high diagnosis accuracy. Especially in early cancer patients, the diagnosis accuracy of the Pan-cancer blood diagnosis marker of the present disclosure is greatly improved compared to that of the traditional method. In addition, the data used in the present disclosure can be obtained only by commonly used whole genome sequencing, and therefore the Pan-cancer blood diagnosis marker of the present disclosure has no special requirements on experimental schemes and sequencing platforms, possesses low operation complexity and low cost, and is suitable for clinical popularization.

Additional aspects and advantages of the present disclosure will be partially given in the following descriptions, which become apparent in part from the following description, or are learned from practice of the present disclosure.

**BRIEF DESCRIPTION OF THE DRAWINGS**

FIG. 1 is a flowchart of a classification method of a sample provided in an embodiment of the present disclosure.
FIG. 2 is a principle diagram of cancer diagnosis in an embodiment of the present disclosure.
FIG. 3 shows a comparison result (A) and an ROC curve (B) of *E-index* calculated by samples in dataset 1 in Example 1 of the present disclosure.
FIG. 4 shows a comparison result (A) an ROC curve (B) of *E-index* calculated by samples in dataset 2 in Example 1 of the present disclosure.
FIG. 5 shows a comparison result (A) and an ROC curve (B) of *E-index* calculated by samples in dataset 3 in Example 1 of the present disclosure.
FIG. 6 shows a comparison result (A) and an ROC curve (B) of *E-index* calculated by stomach cancer and control group samples in dataset 3 in Example 1 of the present disclosure.
FIG. 7 shows a comparison result (A) and an ROC curve (B) of *E-index* calculated by samples in dataset 4 in Example 1 of the present disclosure.
FIG. 8 shows a classification result of samples when a combination of *N-index* and *E-index* for dataset 1 in Example 2 of the present disclosure.
FIG. 9 shows a classification result of samples using a combination of *N-index* and DNA methylation (A) and a combination (B) of *N-index* and *E-index* for dataset 4 in Example 2 of the present disclosure.

## DETAILED DESCRIPTION OF THE EMBODIMENTS

Next, the conception and generated technical effects of the present disclosure will be clearly and completely described in conjunction with embodiments to sufficiently understand the objectives, features and effects of the present disclosure. Obviously, the described embodiments are only some embodiments of the present disclosure, but not all the embodiments. Based on the embodiments of the present disclosure, other embodiments obtained by those skilled in the art without creative efforts are all included within the scope of protection of the present disclosure.

**Next,** the embodiments of the present disclosure will be described in detail, and the described embodiments are illustrative and merely used for explaining the present disclosure, but cannot be understood as limiting the present disclosure.

In the description of the present disclosure, a plurality means one or more, and multiple means two or more. Greater than, less than, exceeding and the like are understood as excluding this number, while above, below, and within are understood as including this number. If it is described that the first and second are only for the purpose of distinguishing technical features, it cannot be understood as indicating or implying relative importance, or implicitly indicating the number of the indicated technical features or implicitly indicating the precedence relationship of the indicated technical features. If a logic sequence is described in the flowcharts, the described or illustrated steps may be executed in a sequence different from that in the flowcharts in some cases.

In the description of the present disclosure, reference terms "one embodiment," "some embodiments," "exemplary embodiments." "examples," "specific examples" or "some examples" refer to specific features, structures, materials or characteristics described in conjunction with the embodiments or examples included in at least one embodiment or example of the present disclosure. In the specification, illustrative expressions of the above-mentioned terms may not necessarily refer to the same embodiments or examples. Furthermore, specific features, structures, materials or characteristics described can be combined in any one or more embodiments or examples in an appropriate manner.

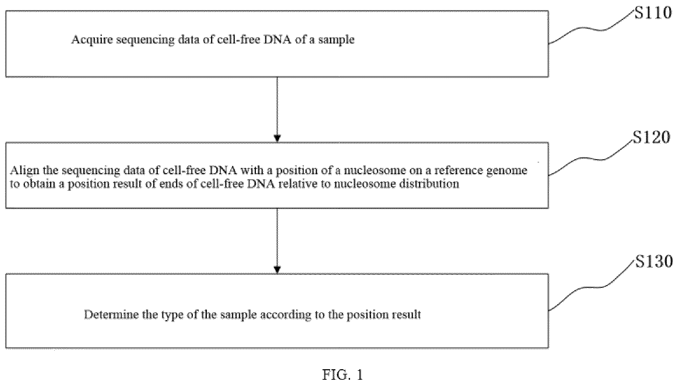

The present disclosure provides a classification method of a sample, referring to FIG. 1. the method including the following steps:

S110, acquiring sequencing data of cell-free DNA from the sample.

Cell-free DNA (cfDNA) refers to a DNA fragment that is free outside a cell, which is generally produced by cell apoptosis or necrosis and released to the outside of the cell. Cell-free DNA is present in various body fluids of an organism, including but not limited to, blood, urine, faeces, cerebrospinal fluid, saliva, pleural effusion, ascites, etc., and therefore the sample in S110 is a sample derived from at least one selected from the above-mentioned sources. In some specific embodiments, the sample includes a blood sample from a specific object, for example at least one selected from whole blood (such as venous whole blood, arterial whole blood and capillary whole blood), plasma, serum, peripheral blood, etc. The sequencing data of cell-free DNA includes sequence data obtained after cell-free DNA is sequenced.

S120, aligning the sequencing data of cell-free DNA with a position of a nucleosome on a reference genome to obtain a position result how the ends of the cell-free DNAs are distributed relative to nucleosomes. S130, determining the type of the sample according to the position result.

The nucleosome is a basic structure unit of a chromatin formed by DNA and histone. The core of each nucleosome is formed by enwinding histone with DNA in a certain number of turns (for example 1.75 tums), and the cores of the nucleosomes is linked through extended linker DNA. Therefore, the position of the nucleosome on the reference genome at least may be a position of DNA enwinding histone in the nucleosome and/or linker DNA between the nucleosomes on the reference genome. Hence, the sequencing data of cell-free DNA is aligned with the position of the nucleosome on the reference genome to finally obtain the position result of the ends of cell-free DNA relative to nucleosome distribution, which actually reflects a relationship of consistency between the ends of cell-free DNA and the distribution of the nucleosomes on the reference genome. In this case, if the ends of cell-free DNA are consistent to the distribution of nucleosomes on the reference genome, it indicates that cell-free DNA is highly likely to come from blood cells: on the contrary, if there is a significant difference between the ends of cell-free DNA and the distribution of the nucleosomes on the reference genome, it indicates that cell-free DNA is highly likely to come from other types of biological cells or tissues except blood cells.

In some specific embodiments, the position of the nucleosome on the reference genome is obtained by at least one method of a)-b):

a) performing sequencing analysis on cells from non-cancer people (or population) to determine the position of the nucleosome of the cell on the reference genome: and
b) analyzing the sequencing data of the cell-free DNA to establish a map of the nucleosome on the reference genome so as to determine the position of the nucleosome on the reference genome.

The cell from the non-cancer population in a) is optionally selected from somatic cells which include, but are not limited to, at least one selected from endothelial cells, epithelial cells, bone cells, chondrocytes, nerve cells, adipocytes, fibroblasts, mesenchymal cells, liver cells, pancreatic islet cells and blood cells. In some specific embodiments, the cell is the blood cell, and the blood cell is a leukocyte. In some specific embodiments, a method for sequencing analysis of the cells from the non-cancer population is at least one selected from Mnase-seq, ChIP-seq, ATAC-seq, NOME-seq, DNase-seq, FAIRE-seq and Cut & Tag. Therefore, the method in a) is actually to perform library construction and sequencing by acquiring DNA and the like in tissue cells, so as to acquire the distribution of nucleosomes in normal cell tissues from the non-cancer population. b) is that a nucleosome map is constructed according to cell-free DNA sequencing data that has been obtained, and then the position of the nucleosome corresponding to cell-free DNA in a sample, for example, refer to a method disclosed in Snyder et al., Cell 2016 Jan 14:164(1-2):57-68. It can be understood that the position information of the nucleosomes on the reference genome can be directly obtained by some existing datasets such as interior datasets that are constructed by other experiments and algorithms and can reflect nucleosome position information of blood cells from a non-cancer population (or a healthy population).

In some specific embodiments, aligning the sequencing data of cell-free DNA with the position of the nucleosome on the reference genome includes: aligning the sequencing data of cell-free DNA with the reference genome to locate cell-free DNA onto the reference genome to acquire the location locus of the end of cell-free DNA on the reference genome; and also includes: acquiring the position information of the nucleosome on the reference genome, and then aligning the location locus of the end of cell-free DNA on the reference genome with the position of the nucleosome on the reference genome.

In some specific embodiments, the reference genome is an optional human reference genome, including but not limited to any one of GRCh36 (hg18), GRCh37 (hg19), GRCh38 (hg38), etc. Of course, the reference genome is not limited thereto, which may also be a specific region on the human reference genome, and a part of regions of interest are used for alignment during the alignment. In addition, genomes of one or more human individuals or specific regions thereon may also be considered.

Therefore, in this step, through the alignment between the sequencing data of cell-free DNA and the reference genome, cell-free DNA is located onto the reference genome to acquire a relative position relationship between the end of cell-free DNA on the reference genome and nuleosome distribution. It can be understood that according to different sequencing methods, the end of cell-free DNA refers to one end or two ends of cell-free DNA. Specifically, when single-end sequencing is conducted, the position of single end on the reference genome is only selected in this step: and when paired-end sequencing is conducted, the end of cell-free DNA selects positions of any one or both of an upstream end and a downstream end on the reference genome. The upstream end and the downstream end refer to an upstream or a downstream of cfDNA during the sequencing. The end and its position usually refer to an end base and a corresponding locus of the base on the reference genome.

In some specific embodiments, the position result of the end of cell-free DNA relative to the nucleosome distribution refers to a fact that the end of cell-free DNA is located in the interior of nucleosome or is located on linker DNA. In some specific embodiments, when the end of cell-free DNA is located in the interior of nucleosome, the sample is a cancer sample; and when the end of cell-free DNA is located on linker DNA, it is determined that the sample is a non-cancer sample. In some specific embodiments, the sample has a plurality of e.g., 10 or more, 100 or more, 1000 or more, 10000 or more and 100000 or more, cell-free DNAs. Therefore, the position result of the ends of cell-free DNAs relative to nucleosome distribution includes: a proportion of ends of cell-free DNAs located in the interior of nucleosome, and/or, a proportion of ends of cell-free DNAs located on linker DNA: and the distribution of ends of cell-free DNAs is reflected by a proportion of ends of total cell-free DNA located in the interior of nucleosome or located on linker DNA.

In some specific embodiments, according to the length of DNA enwinding histone on the core of the nucleosome, different bp conditions are defined as located in the interior of nucleosome. Under normal circumstances, the length of each DNA enwinding histone in each nucleosome is 146 bp, and therefore the middle of the entire length of this DNA fragment is used as the center position of the nucleosome, and an area within 73 bp upstream or downstream of the center position of the nucleosome is defined as the interior of the nucleosome. Of course, it can be understood that the position range of the interior of the nucleosome is not fixed, which may be smaller, for example, within 70 bp, 65 bp. 60 bp, 55 bp, and 50 bp: of course, the range may also be larger, for example within 75 bp, 80 bp, 85 bp, 90 bp. 95 bp, but generally no more than 100 bp. In such cases, a position range on linker DNA is, in direct contrast, a range of greater than 70 bp, 65 bp, 60 bp, 55 bp, 50 bp, or greater than 75 bp, 80 bp, 85 bp, 90 bp, 95 bp and 100 bp.

In some specific embodiments, when a proportion of cell-free DNAs whose ends are located in the interior of nucleosomes in a total amount of cell-free DNAs is greater than a first threshold, and/or, when a proportion of cell-free DNAs whose ends are located on linker DNAs in the total amount of cell-free DNAs is smaller than a second threshold, it is determined that the sample is a cancer sample.

When the proportion of cell-free DNAs whose ends are located in the interior of nucleosomes in the total amount of cell-free DNAs is smaller than or equal to the first threshold, and/or, when the proportion of cell-free DNAs whose ends are located on linker DNAs in the total amount of cell-free DNAs is greater than a second threshold, it is determined that the sample is a non-cancer sample.

When a distance between the ends of a plurality of cell-free DNAs and the center position of the nucleosome is greater than a third threshold, it is determined that the sample is the non-cancer sample; and when the distance between the ends of the plurality of cell-free DNAs and the center position of the nucleosome is smaller than or equal to the third threshold, it is determined that the sample is the cancer sample.

The first threshold and the second threshold can be determined according to practical needs upon meeting requirements as cutoff values, which are for example 0.1, 0.2, 0.3, 0.4, 0.5, 0.6, 0.7, 0.8, 0.9, etc. The same applies to the third threshold, and the third threshold, for example, can be 50 bp, 55 bp, 60 bp, 65 bp. 70 bp. 73 bp, 75 bp, 80 bp. 85 bp. 90 bp, 95 bp, 100 bp, etc.

In some embodiments, a specific parameter for describing a position result is given.

In some embodiments, the specific parameter is *N-index,* which meets

$$N - index = \frac{N_{\text{interior of nucleosome}}}{N_{\text{all}}}$$

where $N_{\text{interior of nucleosome}}$ is the number of cell-free DNAs whose ends are located in the interior of nucleosomes in a plurality of cell-free DNAs: and $N_{\text{all}}$ is the total number of the plurality of cell-free DNAs. In some embodiments, the sample is a cancer sample when *N-index* is greater than a first cutoff value: the sample is a non-cancer sample when *N-index* is smaller than or equal to the first cutoff value; and the cancer sample is a sample of at least one type of cancer of liver cancer, lung cancer, intestinal cancer, pancreatic cancer, bile duct cancer, breast cancer and ovarian cancer.

In some embodiments, the specific parameter is *N'-index,* which meets

$$N' - index = \frac{N_{\text{interior of nucleosome}}}{N_{\text{linker DNA}}}$$

where $N_{\text{linker DNA}}$ is the number of cell-free DNAs whose ends are located on linker DNAs between the nucleosomes. In some embodiments, the sample is a non-cancer sample when *N'-index* is greater than a second cutoff value; and the sample is a cancer sample when *N'-index* is smaller than or equal to the second cutoff value.

In some embodiments, the specific parameter is *N"-index,* which meets

$$N'' - index = \sum_{i=1}^{n} a_i b_i$$

where $n$ is the number of ends in the plurality of cell-free DNAs, $a_i$ is a distance between the *i*-th end and the center of the nucleosome, and $b_i$ is a weight of the i-th end. In some embodiments, the sample is a non-cancer sample when *N"-index* is greater than the third cutoff value: and when the sample is a cancer sample when *N"-index* is smaller than or equal to the third cutoff value.

In some embodiments, normalization may also be further performed on *N-index* or *N'-index*. It can be understood that in this step, the results are compared by using an appropriate statistical method in addition to the weighted model, thereby judging their consistency, so as to determine the enrichment of cfDNA fragment ends in a nucleosome region.

The specific sizes of the above-mentioned first cutoff value and second cutoff value are similar to the thresholds as described above, which can be determined according to practical needs upon meeting the requirements as cutoff values, for example. 0.1, 0.2. 0.3, 0.4, 0.5. 0.6, 0.7. 0.8, 0.9. 1.0. 1.1. 1.2, 1.3. 1.4, 1.5, 1.6, 1.7, 1.8, 1.9, 2.0, 2.5, 3.0, etc. The same applies to the third cutoff value, for example, when a weighted mean is calculated, the third cutoff value can be 50 bp, 55 bp, 60 bp, 65 bp, 70 bp, 73 bp, 75 bp, 80 bp. 85 bp. 90 bp. 95 bp, 100 bp, etc.

In some specific embodiments, cell-free DNA and a nucleosome are cell-free DNA and a nucleosome at a target genome region. The target genome region can be a human whole genome, or a designated region of the human whole genome, for example, a part of chromosomes such as any one or more of chr1-22, chrX and chrY, or one or more custom regions in the human whole genome. It is noted that if the designated region in the human whole genome is used, it can be achieved by at least one or more of the following methods, for example, a capture or enrichment technique (such as target capture and whole exome sequencing) is used during the sequencing before data acquisition, or sequencing read at the designated region is only selected during the data acquisition. The reference genome and the target genome region can be the same or different, however, obviously, both of them need to have overlapped parts to complete the construction of the model, and in some cases, the reference genome covers the target genome region.

For the above-mentioned classification method of the sample, it is mainly based on the following conception: cell-free DNA is mostly produced by degradation of chromatin during the cell apoptosis, the fragmentation pattern of cell-free DNA is affected by nucleosome distribution: the ends of cell-free DNA tend to fall on linker DNA between nucleosomes, while there are few of ends of cell-free DNA located in the interior of nucleosome. In non-cancer patient samples, cfDNA mostly comes from blood cells, its fragmentation pattern is highly consistent with nucleosome distribution in blood cells: but in solid tumor cancer patients, tumor and para-carcinoma tissues do not belong to blood cells, their nucleosome distribution pattern is different from that in blood cells, and therefore there is relatively low consistency between DNA released by the tumor and para-carcinoma tissues and nucleosome distribution in blood cells, leading to reduction in consistency between the fragmentation pattern of cell-free DNA in peripheral blood and nucleosome distribution in blood cells. Therefore, the consistency between the fragmentation pattern of cell-free DNA in peripheral blood and nucleosome distribution in blood cells can be used as a potential Pan-cancer diagnosis marker. And then, in this scheme, samples are classified by quantifying the consistency between a position distribution pattern of ends of cfDNA and a distribution pattern of normal nucleosomes, for example, a proportion of ends of cell-free DNAs located in the interior of nucleosomes in blood cells.

It can be understood that in the classification method of the sample provided in embodiments of the present disclosure, various types of cancers can be distinguished. The specific type of the cancer can be at least one selected from solid tumors including brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphoma and skin cancer. Among them, the brain cancer includes primary including neurogliomas and non-neurogliomas (such as meningioma, neuroblastoma, medullary pituitary adenomas, primary CNS lymphoma, and central nervous system germ cell tumors) and secondary brain cancer including malignant tumors that originate elsewhere but have spread (metastasized) to the brain, such as breast cancer and lung cancer that have been metastasized to the brain: head and neck cancer, including oral/oropharyngeal cancer, nasopharyngeal cancer, nasal/sinus cancer, laryngeal/pharyngeal cancer, salivary gland cancer, etc.: lung cancer, including small cell lung cancer, non-small cell lung cancer, etc.; liver cancer, including hepatocellular carcinoma, bile duct cancer, hepatoblastoma, etc.; breast cancer, including carcinoma in situ (such as lobular carcinoma in situ and ductal carcinoma in situ), invasive breast cancer (invasive lobular carcinoma, invasive ductal carcinoma), etc.: stomach cancer, including gastric adenocarcinoma, gastrointestinal stromal tumor, gastrointestinal leiomyosarcoma, gastrointestinal carcinoid, gastrointestinal lymphoma, etc.; cervical cancer, including squamous cell carcinoma and adenocarcinoma, etc.: ovarian cancer, including epithelial tumors, embryonal tumors, stromal tumors, etc.: pancreatic cancer, including pancreas cancer, cystic tumors, acinar cell carcinoma, sarcoma and ampullary carcinoma, etc.; thyroid cancer, including papillary thyroid carcinoma, follicular thyroid carcinoma. Hirschsprung's cell carcinoma, etc.; lymphoma, including Hodgkin's lymphoma, non-Hodgkin's lymphoma, etc.: and skin cancer, including non-melanoma skin cancer and melanoma skin cancer, etc.

In some specific embodiments of the present disclosure, the sample is a blood sample, and the nucleosome is a nucleosome in blood cells. In some specific embodiments, the blood cell can be one or more types of blood cells, such as at least one selected from lymphocytes (such as T lymphocytes and B lymphocytes), neutrophils, cosinophils, basophils, and monocytes.

When the technical solution required in the present disclosure is adopted, commonly used whole genome shotgun sequencing is used in an experiment, with simple experimental operation and low requirements on initial raw materials: a diagnosis model can utilize most of data information, has low requirements on sequencing depth, is suitable for single-end sequencing, can further reduce experimental cost, and is low in diagnosis cost. In addition, the present disclosure also has the following features:

1) the Pan-can blood diagnosis marker of the present disclosure only requires cell-free DNA sequencing data without analysis of DNA modification (such as DNA methylation), but in DNA methylation sequencing data, cell-free DNA sequencing data is combined with DNA methylation to improve accuracy.
2) The Pan-can blood diagnosis marker of the present disclosure can be used in various types of cancer (i.e.. Pan-cancer cancer diagnosis is achieved).
3) The Pan-can blood diagnosis marker of the present disclosure has no special requirements on chemical reagents and sequencing platforms used in experiments (strong universality).

In addition, it is noted that in some embodiments, the classification method provided in the present disclosure only can exclude the possibility of classifying some samples as "non cancer," and cannot determine the type of cancer. Cancer diagnosis and tumor tracing belong to two different categories, and their applicable objects are also different. A Pan-cancer classification method provided in embodiments of the present disclosure is especially suitable for populations participating in routine physical examinations, and patients with early-stage cancers are screened by cfDNA analysis. After the classification provided in the present disclosure is completed, it is possibly necessary to further trace the tumor to determine the specific type of cancer.

Based on this, the classification method further includes a step of verifying a cancer source to further classify the sample so as to complete cancer tracing, or the classification method is combined with other classification methods to further improve the accuracy for cancer diagnosis. Specifically, analysis is performed in combination with other data of cell-free DNA or other types of data from a subject. Other data of cell-free DNA include, but are not limited to, copy number variation data of cell-free DNA, methylation data of cell-free DNA. length distribution data of cell-free DNA, end base distribution data of cell-free DNA, end distribution consistency data of cell-free DNA, etc., and the other types of data from the subject may be marker data and image data, etc., the above-mentioned data are analyzed through a model, and then the type of the cancer is determined in combination with an analysis result and a comparison result. In some specific embodiments, the end distribution consistency data of cell-free DNA refers to comparison of the consistency between the occurrence frequency of different loci in a target genome region as ends of cell-free DNA from a sample and the occurrence frequency of different loci in the target genome region as ends of cell-free DNA from a non-cancer population. If the consistency is relatively high, the risk of suffering from cancer is relatively low: and if the consistency is low, the risk of suffering from cancer is relatively high. For example, the end distribution consistency of cell-free DNA is calculated by consistency *E-index:*

$$E - index - \frac{1}{N_{sam}} \sum_{i=1}^{n} x_i y_i$$

where n is the number of loci occurring in an end distribution model, $x_i$ is the frequency of the locus i occurring in an end distribution model, $y_i$ is the frequency of the locus i occurring in ends of cell-free DNA from a sample: and *N* sample is the total number of cfDNA in a sample. Specifically, refer to prior application 202210496595.9.

It can be understood that when multiple methods are combined to perform cancer tracing or improve the accuracy of diagnosis, it is not necessary to follow a classification rule under a single method after combination, or a unique rule after combination can also be formed, which needs to be specifically judged according to methods used for practical combination and is not described in detail here. The classification of the sample is that subjects who belong to samples are classified into a population with cancers or a population without cancers, further may be classified into a population with a specific cancer, or a population with metastasis of cancers, for example liver metastasis of lung cancer, etc. The marker refers to a small molecule (such as amino acids, cholines and the like as metabolic markers), a nucleic acid (such as miRNA, mRNA and lncRNA), a protein, a flora, etc. that are related to one or some specific cancer types. It can be understood that in the embodiments of the present disclosure, marker data is data of other types of markers that are unrelated to copy number variation, methylation, length distribution, end base distribution and end distribution of cell-free DNA as described above, or data using other quantification methods of cell-free DNA as markers.

The present disclosure further provides a computer-readable storage medium, storing computer-executable instructions which, when executed by a computer, cause the computer to implement the classification method described above.

The present disclosure further provides a device, including a processor and a memory, where the memory stores a computer program runnable on the processor which, when executed by the processor, causes the processor to implement the above-mentioned classification method.

As a non-transient computer-readable storage medium, the memory is configured for storing non-transient software programs and non-transient computer executable programs, such as the previous classification method described in the embodiments of the present disclosure. The processor operates the non-transient software programs and instructions stored in the memory so as to achieve the classification of the sample.

The memory may include a program storage area and a data storage area, wherein, the program storage area stores an operating system and an application required for at least one function: and the data storage area stores and executes the above-mentioned programs. In addition, the memory may include a high-speed random access memory, and may also include a non-transient memory such as at least one disk storage device, a flash memory device, or other non-transient solid-state storage devices.

In some embodiments of the present disclosure, the memory optionally includes memories remotely set relative to the processor, and these remote memories are connected to the processor through a network. The examples of the above-mentioned network include, but are not limited to, Internet, Intranet. LAN, a mobile communication network and a combination thereof.

The non-transient software programs and instructions required for achieving the above-mentioned evaluation are stored in the memory. When the non-transient software programs and instructions are executed by one or more processors, the classification of the sample as described above is executed.

The present disclosure further provides a system for diagnosing a cancer, the system for diagnosing the cancer including:

an acquisition module for acquiring sequencing data of cell-free DNA from a sample: and

a comparison module for aligning the sequencing data of the cell-free DNA with a position of a nucleosome on a reference genome to obtain the position result of the end of the cell-free DNA relative to nucleosome distribution, and judging whether the sample is a cancer sample according to the position result.

The specific implementation mode of the comparison module refers to corresponding steps in the previous classification method.

It can be understood that the system for diagnosing the cancer can also include other related modules, or a corresponding acquisition module and comparison module have multiple functions.

The device or system embodiments as described above are merely illustrative, modules serving as separating parts may be or may not be physically separated, i.e., may be located in one place, or distributed on multiple network units. A part or all of the modules can be selected according to practical needs to achieve the purpose of embodiments of the present disclosure.

It can be understood that all or same steps and the like disclosed hereinabove may be implemented as software, firmware, hardware and an appropriate combination thereof. Some physical components or all physical components may be implemented as software executed by processors such as a central processing unit, a digital signal processor or a microprocessor, or implemented as hardware, or implemented as an integrated circuit such as an application specific integrated circuit. Such the software can be distributed on the computer-readable medium. The computer-readable medium may include a computer storage medium (or non-transient medium) and a communication medium (or a transient medium). It can be understood that the computer storage medium includes volatile and non-volatile, removable and non-removable media that are implemented in any method or technology for storing infonnation (such as computer-readable instructions, data structures, program modules, or other data). The computer storage medium includes, but is not limited to, a random access memory (RA), a read only memory (ROM), an electrically erasable programmable read-only memory (EEPROM), a flash memory or other memory technologies, a compact disc read-only memory (CD-ROM), a digital video disk (DVD) or other optical disk storage, magnetic box, magnetic tape, magnetic disk storage or other magnetic storage devices, or any other media that can be used to store desired information and accessed by a computer.

In addition, it can be understood that the communication medium typically contains computer-readable instructions, data structures, program modules, or other data in modulated data signals such as carriers or other transmission mechanisms, and may include any information delivery media.

The present disclosure further provides a Pan-cancer blood diagnosis marker. The Pan-cancer blood diagnosis marker includes at least one selected from *N-index* and *N'-index* that characterize the consistency between a fragmentation pattern of cell-free DNA and nucleosome distribution in blood cells, which meets

$$N - index = \frac{N_{\text{interior of nucleosome}}}{N_{\text{all}}}$$

wherein, $N_{\text{interior of nucleosome}}$ is the number of cell-free DNAs whose ends are located in the interior of nucleosomes in a plurality of cell-free DNAs: $N_{\text{all}}$ is the total number of the plurality of cell-free DNAs:

$$N' - index = \frac{N_{\text{interior of nucleosome}}}{N_{\text{linker DNA}}}$$

wherein, $N_{\text{linker DNA}}$ is the number of cell-free DNAs whose ends are located on linker DNAs between the nucleosomes:

$$N'' - index = \sum_{i=1}^{n} a_i b_i$$

wherein, $n$ is the number of ends of a plurality of cell-free DNAs, $a_i$ is a distance between an $i$-th end and the center of the nucleosome, and $b_i$ is a weight of the $i$-th end.

The present disclosure further provides use of the above-mentioned Pan-cancer blood diagnosis market or a product for testing the Pan-cancer blood diagnosis marker in preparation of cancer diagnosis products.

The classification method of the present disclosure will be described in combination with specific embodiments.

Example 1

1. Source of sample

24 volunteers were recruited from a physical examination population in a hospital. Through the physical examination result, it was verified that there were no cancer patients. Before sample collection, the 24 volunteers all signed infonned consent form and underwent ethical review.

2. Collection and treatment of peripheral blood

Peripheral blood (6 ml) was collected from a vein using a blood collection tube containing an anticoagulant (ethylene diamine tetraacetic acid (EDTA) and centrifuged for 15 min using a centrifugal machine under the conditions of 4°C and 1600×g, an upper-layer liquid was sucked and transferred to a new centrifugal tube and then centrifuged for 15 min using the centrifugal machine under the conditions of 4°C and 16000×g, then an upper-layer liquid (i.e., plasma) was sucked, transferred to a cryopreservation tube and stored in a refrigerator at -80°C.

3. Extraction and sequencing of cfDNA

cfDNA was extracted from plasma using a commercially available kit and device, a sequencing library was constructed, and high-throughput sequencing (paired ends) was conducted. Sequencing linkers, low-quality cycles and repetitive sequences were removed from sequencing raw data, then the rest data were aligned to a human whole genome, and finally repetitive sequences and low-quality alignment results were removed to obtain a final alignment result. The positions of two ends of each cfDNA on the genome were extracted. i.e., end locus information of cfDNA.

4. Acquisition of nucleosome distribution data in blood cells

In this example. Mnase-seq experimental data of a GM12878 cell line were used. After nucleosome distribution data in blood cells were acquired, and 73 bp upstream or downstream of the center position of the nucleosome were defined as a nucleosome protecting area.

5. Calculation method of consistency (N-index) between ends of cfDNA end and nucleosome distribution

For each to-be-tested sample, the number $N_{\text{interior of nucleosome}}$ of fragment ends located in the interior of a nucleosome protecting area defined in the previous step in a target genome region and the number $N_{\text{all}}$ of cfDNA fragment ends in this region were analyzed, and $N_{\text{interior of nucleosome}}$ was divided by $N_{\text{all}}$ to obtain the N-index value defined here.

$$N - index = \frac{N_{\text{interior of nucleosome}}}{N_{\text{all}}}$$

The target genome area studied here was a whole genome range or a partial chromosome region that was artificially screened, they could achieve similar effects. For paired-end sequencing data, there were many calculation methods, for example, $N_{\text{interior of nucleosome}}$ was calculated as 1 or 0 if only one end is located in the interior of nucleosome, $N_{\text{interior of nucleosome}}$ was calculated as 1 or 2 if both of two ends are located in the interior of nucleosome, however, the calculation method should remain fixed within the dataset, so that quantified results (i.e., N-index) were compared between samples.

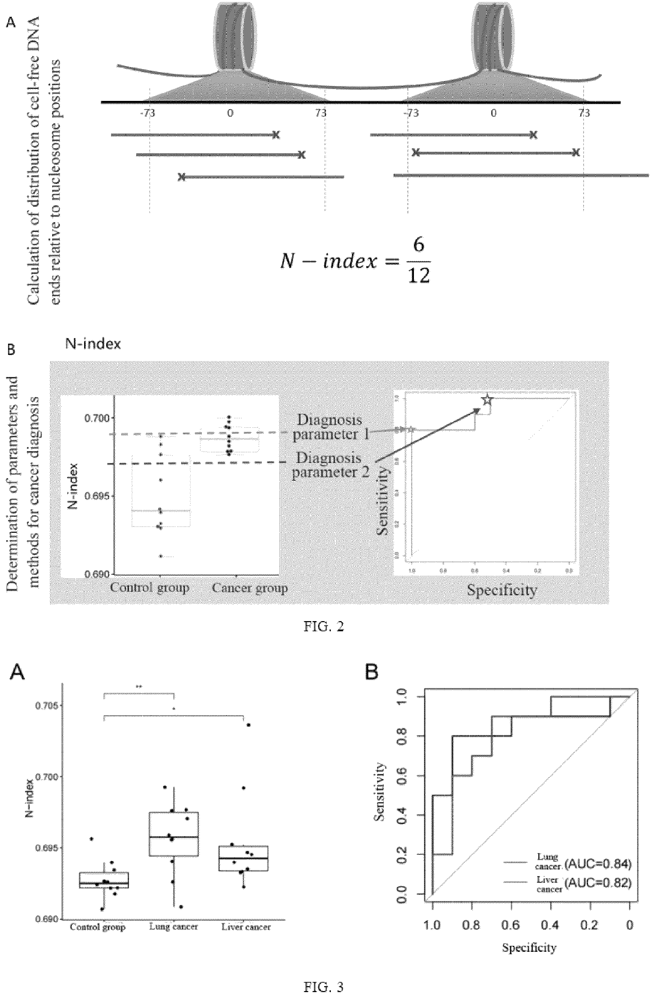

Referring to A in FIG. 2. there were 6 cfDNAs and 2 nucleosomes in the target genome region. If paired-end sequencing was used, there were 12 ends of which 6 ends fallen into the interior of the nucleosome protecting area: and therefore, $N_{\text{interior of nucleosome}}$ was 6, $N_{\text{all}}$ was 12, and N-index=6/12=1/2.

6. Cancer diagnosis method

Cancer diagnosis parameters were first determined. A non-cancer control sample set and a cancer patient set (which was a single cancer type, or multiple cancer types) were constructed, and an N-index value of each sample cfDNA was respectively calculated. Since there was tumor-derived cfDNA in cancer patients, as a whole, the N-index value of the cancer patient should be higher than that of the non-cancer patient, and therefore a cutoff value (i.e., diagnosis parameter) was used for distinguishing, so as to achieve cancer diagnosis.

A receiver operating characteristic curve (ROC) was used to evaluate indicators such as accuracy, sensitiveness and specificity of cancer diagnosis using different cutoff values; and an Area Under the Curve (AUC) value was used to evaluate the overall effect of the diagnosis method: the higher the AUC (the maximum was 1), the better the effect. Finally, the cutoff value was determined according to actual demands, for example community screening may require specificity>99%, while high-risk populations may require sensitivity>95%.

After the cutoff value was determined, the *N-index* value of cfDNA of each to-be-tested patient was calculated. If the *N-index* value was higher than the cutoff value, the patient was a cancer patient, or else, the patient is a non-cancer patient. Alternatively, cancer diagnosis was conducted by using the *N-index* value as a characteristic value in combination with other information (such as image data, a cfDNA methylation level and length distribution).

Referring to B in FIG. 2. under the condition of sufficient sample sizes, the difference between *N-index* values of a cancer group and a control group has a certain statistical significance. In this case, a specific diagnosis parameter was used as the cutoff value to serve as a distinguishing boundary line for two groups of samples, and an appropriate cutoff value was selected according to different requirements such as specificity and sensibility.

The samples were classified by respectively using different sources of datasets. The results were as follows:

(1) A dataset 1 contained sample data from 10 non-cancer patients (control group), 10 liver cancer patients and 10 lung cancer patients (the source of the dataset 1 refers to Liang et al. Whole-genome sequencing of cell-free DNA yields genome-wide read distribution patterns to track tissue of origin in cancer patients. Clin Transl Med. 2020 Oct: 10(6):e177.).
*N-index* values of these patients were calculated according to an *N-index* calculation method provided previously. The results are as shown in A of FIG. 3. the *N-index* values of cancer patients in 2 groups were both greater than those of non-cancer patients as a whole. Referring to B of FIG. 3, ROC analysis showed that *N-index* effectively distinguished cancer patients from non-cancer patients, AUC values in liver cancer patients reached 0.82, while AUC values in lung cancer patients reached 0.84, and P values were all less than 0.05. When specificity was required as 0.8, sensitivities were respectively 0.8 (lung cancer) and 0.7 (lung cancer).

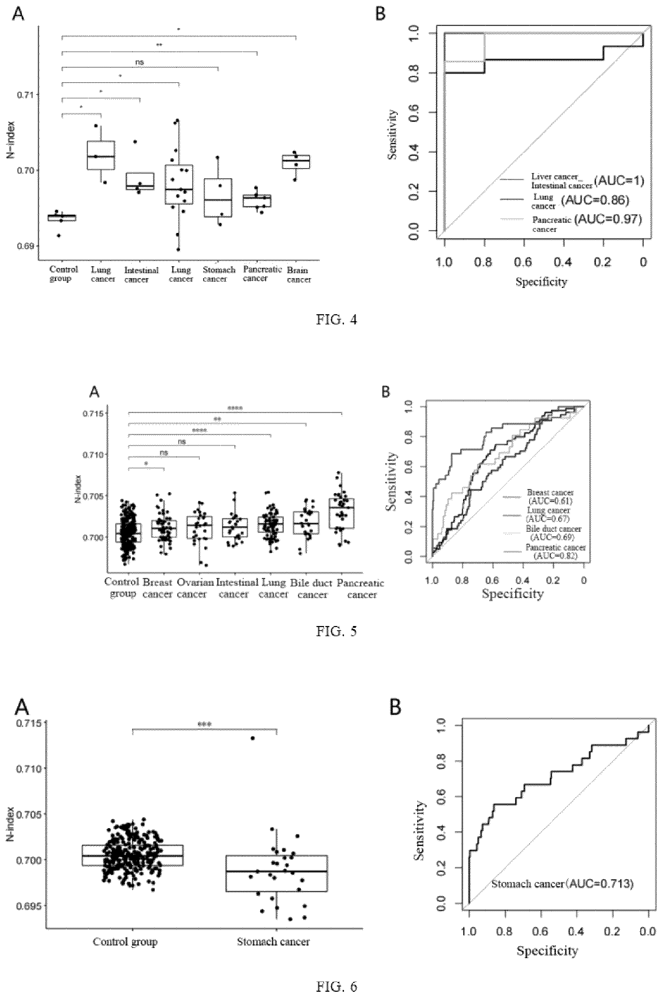

(2) A dataset 2 contained sample data from 4 non-cancer patients. 4 brain tumor patients. 4 colorectal cancer patients, 4 stomach cancer patients. 3 liver cancer patients. 15 lung cancer patients and 7 pancreatic cancer patients (the source of the dataset refers to Song et al. 5-Hydroxymethylcytosine signatures in cell-free DNA provide information about tumor types and stages. Cell Res. 2017 Oct; 27(10):1231-1242., and a part of cancer samples were selected).
*N-index* values of these patients were calculated according to an *N-index* calculation method provided previously. The results are as shown in A of FIG. 4. the *N-index* values of these cancer patients, except stomach cancer patients, were all greater than those of non-cancer patient control groups. Referring to B of FIG. 4, pancreatic cancer patients, lung cancer patients and liver cancer or colorectal cancer patients (7 patients in total) were analyzed. The result showed that cancer diagnosis was conducted by using *N-index,* AUC values were respectively 1.0 (liver cancer or colorectal cancer). 0.86 (lung cancer) and 0.97 (pancreatic cancer), and P values were all less than 0.05; when specificity was required as 0.8. sensitivities were respectively 1.0 (liver cancer or colorectal cancer), 0.86 (lung cancer) and 1.0 (pancreatic cancer).

(3) A dataset 3 contained sample data from 231 non-cancer patients, 26 bile duct cancer patients, 54 breast cancer patients, 27 colorectal cancer patients, 27 stomach cancer patients, 35 pancreatic cancer patients. 79 lung cancer patients and 28 ovarian cancer patients (the source of the dataset refers to Cristiano et al. Genome-wide cell-free DNA fragmentation in patients with cancer. Nature 2019; 570:(385-389)., and a part of cancer samples were selected from therein).

*N-index* values of these patients were calculated according to an *N-index* calculation method provided previously. The results are as shown in A of FIG. 5. in these cancer patients, except ovarian cancer and intestinal cancer patients, the *N-index* values of other groups were all greater than those of non-cancer patient control groups as a whole. The ROC analysis results are as shown in B of FIG. 5, indicating that the AUC values of *N-index* for cancer diagnosis in these cancer types were between 0.61 and 0.82, and the P values were all less than 0.05.

Referring to A and B of FIG. 6, different from manifestations of other cancer types, the *N-index* of stomach cancer patients was down-regulated and matched with a Song dataset, and ROC analysis showed that the AUC value was 0.713, and the P value was less than 0.05.

(4) A dataset 4 contained sample data from 37 non-cancer patients and 8 liver cancer patients (the source of the dataset refers to Zhang, et al. Hypomethylation in HBV integration regions aids non-invasive surveillance to hepatocellular carcinoma by low-pass genome-wide bisulfite sequencing. BMC Medicine 2020. Aug 3:18(1):200.).

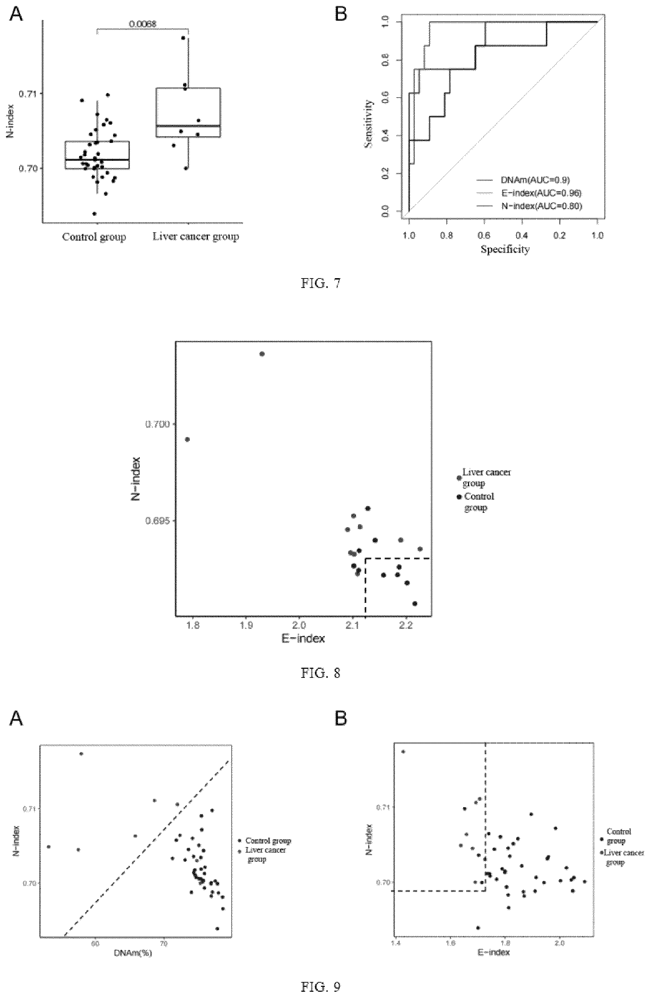

*N-index* values of these patients were calculated according to an *N-index* calculation method provided previously. The results are as shown in A of FIG. 7, the *N-index* values of liver cancer patients were greater than those of non-cancer patients. ROC analysis results are as shown in B of FIG. 7. indicating that *N-index* can effectively distinguish liver cancer patients from non-cancer patients, the AUC values reached 0.80, and the P values were all less than 0.05.

It can be seen in combination with the above-mentioned results that the cancer marker provided in examples of the present disclosure do not need modeling, and can be used on different sources of datasets, with good universality.

In addition, it is noted that distribution data of nucleosomes in blood cells in Example 1 used Mnase-seq experimental data of a GM12878 cell line. As a B lymphocyte line, the GM12878 cell line depends on nucleosome distribution of leukocytes, which only can roughly stimulate a true source of cell-free DNA in human plasma as a whole.

Example 2

In this example, classification was conducted in combination with *N-index* and *E-index* as well as DNA methylation data through cutoff values. In terms of performance indicators, the most clinically meaningful indicator was used, i.e., sensitivity when specificity was 100% (for large-scale screening of asymptomatic patients), or specificity when sensitivity was 100% (for precise diagnosis of symptomatic patients).

The steps of a classification method of a sample by adopting *E-index* were as follows:

I. Construction of cancer diagnosis model

1. Source of sample

Volunteers were recruited from a physical examination population in a hospital. Through the physical examination result, it was verified that there were no cancer patients. Before sample collection, the volunteers all signed informed consent form and underwent ethical review.

2. Collection and treatment of peripheral blood

Peripheral blood (6 ml) was collected from a vein using a blood collection tube containing an anticoagulant (EDTA) and centrifuged for 15 min using a centrifugal machine under the conditions of 4°C and 1600×g, an upper-layer liquid was sucked, transferred to a new centrifugal tube and then centrifuged for 15 min using the centrifugal machine under the conditions of 4°C and 16000 ×g, then an upper-layer liquid was sucked (i.e., plasma) and transferred to a cryopreservation tube to be stored in a refrigerator at -80°C.

3. Extraction and sequencing of cfDNA

cfDNA was extracted from plasma using a commercially available kit and device, a sequencing library was constructed, and high-throughput sequencing (paired ends) was conducted. Sequencing linkers, low-quality cycles and repetitive sequences were removed from sequencing raw data, then the rest data were aligned to a human whole genome, and finally repetitive sequences and low-quality alignment results were removed to obtain a final alignment result. The positions of two ends of each cfDNA on the genome were extracted, i.e., end locus information of cfDNA. Referring to FIG. 3, the frequency for locating the end of cfDNA corresponding to each locus on the reference genome to the locus was counted to obtain a statistical table for the frequency of each locus in the reference genome serving as the end of cfDNA, so as to construct a locus-frequency end distribution model.

II. Sample classification

1. Calculation of cfDNA end distribution consistency (*E-index*)

For each to-be-tested sample, information of cfDNA ends was extracted according to a method for constructing a cancer diagnosis model, and the total number ($N_{sample}$) of cfDNA fragments in the alignment result was recorded.

For all loci in a genome, the frequency of using the end of cfDNA in the end distribution model was used as a weight, and the frequency of using the end of cfDNA in the to-be-tested sample was weighted; then the weighted values were normalized using $N_{sample}$, and the normalized value was used as *E-index* characterizing the consistency between cfDNA end distribution in the to-be-tested sample and a non-cancer population, referring to the following formula:

$$E - index - \frac{1}{N_{sample}} \sum_{i=1}^{n} x_i y_i$$

wherein, n is the number of loci occurring in an end distribution model, $x_i$ is the frequency of occurrence of the locus i in the end distribution model, and $y_i$ is the frequency of occurrence of the locus i in the ends of cell-free DNA of a sample; and $N$ is the total number of cfDNAs in the sample.

The determination of the subsequent cutoff value and the classification method of the sample refer to those in Example 1.

A method for classifying a sample through DNA methylation data refers to Chan et al. PNAS 2013 Nov 19: 110(47):18761-8.

Referring to FIG. 8. in a liver cancer sample of dataset 1. if cancer prediction was conducted by using *N-index* alone, the specificity was 40% when the sensitivity was 100%. If a combination of *N-index* and *E-index* was used, the specificity was improved to 50% when the sensitivity was 100%.

Referring to B of FIG. 7. in a dataset 4, *N-index, E-index* and DNA methylation all effectively distinguished liver cancer patients from non-cancer patients. If cancer prediction was conducted by using *N-index* alone, the sensitivity was 37.5% when the specificity was 100%: as shown in A of FIG. 9. if a combination of *N-index* and DNA methylation was used, the sensitivity was improved to 75% when the specificity was 100%: if cancer prediction was conducted by using *N-index* alone, the specificity was 21.62% when the sensitivity was 100%: and as shown in B of FIG. 9, if a combination of *N-index* and *E-index* was used, the specificity was improved to 91.89% when the sensitivity was 100%

Example 3

This example provides a system for diagnosing a cancer, the system including:

> an acquisition module for acquiring sequencing data of cell-free DNA of a sample: and
> a comparison module for aligning the sequencing data of the cell-free DNA with a position of a nucleosome on a reference genome to obtain a position result of the end of the cell-free DNA relative to nucleosome distribution, and judging whether the sample is a cancer sample according to the position result.

Meanwhile, during the comparison in the comparison module, calculation and comparison were conducted through *N''-index*:

$$N'' - index = \sum_{i=1}^{n} a_i b_i$$

wherein, $n$ is the number of ends of cell-free DNA, $a_i$ is a distance between an i-th end and the center of the nucleosome, and $b_i$ is a weight of the i-th end. Meanwhile, distances different from the center of the nucleosome were given to weights with different sizes.

The system for diagnosing the cancer achieves the cancer diagnosis effect similar to that in a separate dataset in Example 1, which is not described in detail here.

Example 4

This example provides a system for diagnosing a cancer. During the use, the system is different from that in Example 3 in that the position of the nucleosome is subjected to sequencing analysis using whole blood samples from healthy people to determine the position of the nucleosome on a reference genome.

The system for diagnosing the cancer achieves the cancer diagnosis effects similar to those in different datasets in Example 1, without detailed description here.

Example 5

This example provides a system for diagnosing a cancer. During the use, the system is different from that in Example 3 in that the position of the nucleosome is subjected to sequencing analysis using whole blood samples from healthy people when being aligned to determine the position of the nucleosome on a reference genome in epithelial cells.

The system for diagnosing the cancer achieves the cancer diagnosis effects similar to those in different datasets in Example 1, which is not described in detail here.

Based on the above-mentioned examples, it can be seen that the classification method and diagnosis markers provided in the present disclosure are used for various types of cancer, with high universality and wide application scenarios. Meanwhile, the above-mentioned method is relatively high in diagnosis accuracy, especially for early cancer patients, the diagnosis accuracy of the above-mentioned method is significantly improved compared with that in the traditional method. In addition, only commonly used whole genome sequencing is needed to obtain data used for achieving the above-mentioned method. The above-mentioned method has no special requirements on experimental schemes and sequencing platforms, and is low operating complexity, low in cost and suitable for clinical popularization.

Detailed description was made to the present disclosure in combination with embodiments, but the present disclosure is not limited to the above-mentioned embodiments, and various changes can also be made without departing from the purpose of the present disclosure in the scope of knowledge possessed by persons of ordinary skill in the art. Moreover, in case of no conflict, embodiments of the present disclosure and features in embodiments can be combined with each other.

**Claims**

1. A classification method of a sample, comprising the following steps:
acquiring sequencing data of cell-free DNAs from the sample:

aligning the sequencing data of the cell-free DNAs with the positions of nucleosomes on a reference genome, to obtain a position result how ends of the cell-free DNAs are distributed relative to nucleosomes: and
determining a type of the sample according to the position result:

preferably, the end of the cell-free DNA comprises at least one selected from an upstream end and a downstream end;
preferably, the reference genome comprises a human whole genome or a designated region of the human whole genome: and
preferably, the sample is a blood sample.

2. The classification method according to claim 1, wherein the position result how ends of the cell-free DNAs are distributed relative to nucleosomes comprises:
the ends of the cell-free DNAs are located in the interior of nucleosomes, or are located on linker DNAs:

preferably, determining the type of the sample according to the position result comprises:

determining that the sample is a cancer sample when the ends of the cell-free DNAs are located in the interior of nucleosomes: and
determining that the sample is a non-cancer sample when the ends of the cell-free DNAs are located on linker DNAs:

preferably, the interior of a nucleosome is within 100 bp upstream or downstream of the center position of the nucleosome:
preferably, the interior of a nucleosome is within 95 bp upstream or downstream of the center position of the nucleosome;
preferably, the interior of a nucleosome is within 90 bp upstream or downstream of the center position of the nucleosome;
preferably, the interior of a nucleosome is within 85 bp upstream or downstream of the center position of the nucleosome:

preferably. the interior of a nucleosome is within 80 bp upstream or downstream of the center position of the nucleosome:
preferably, the interior of a nucleosome is within 75 bp upstream or downstream of the center position of the nucleosome:

preferably, the interior of a nucleosome is within 73 bp upstream or downstream of the center position of the nucleosome;
preferably, the cancer is a solid tumor:

preferably, the cancer is at least one selected from brain cancer, head and neck cancer, lung cancer, liver cancer, breast cancer, stomach cancer, colon cancer, rectal cancer, cervical cancer, ovarian cancer, pancreatic cancer, prostate cancer, thyroid cancer, lymphatic cancer and skin cancer.
preferably, the positions of nucleosomes on the reference genome are obtained by at least one method from a)-b):

a) performing sequencing analysis on cells from a non-cancer patient to determine the positions of the nucleosomes on the reference genome: and
b) analyzing the sequencing data of the cell-free DNAs to establish a map of the nucleosomes on the reference genome, so as to determine the positions of the nucleosomes on the reference genome;

preferably, the cell is optionally selected from somatic cells which are at least one selected from endothelial cells, epithelial cells, bone cells, chondrocytes, nerve cells, adipocytes, fibroblasts, mesenchymal cells, liver cells, pancreatic islet cells and blood cells:

preferably, the cell is the blood cell, and the blood cell is a leukocyte:
preferably, a method for sequencing analysis of the cells from the non-cancer patient is at least one selected from Mnase-seq, ChIP-seq, ATAC-seq, NOME-seq, DNase-seq, FAIRE-seq and Cut & Tag.

3. The classification method according to claim 2. wherein the position result how ends of the cell-free DNAs are distributed relative to nucleosomes comprises: a proportion of ends of a plurality of the cell-free DNAs located in the interior of nucleosomes, and/or a proportion of the ends of the plurality of the cell-free DNAs located on linker DNAs: preferably, determining the type of the sample according to the position result comprises: determining that the sample is a cancer sample when a proportion of the plurality of the ends of the cell-free DNAs located in the interior of nucleosomes is greater than a first threshold, and/or, when a proportion of the ends of the plurality of the cell-free DNAs located on linker DNAs is smaller than a second threshold:

determining that the sample is a non-cancer sample when the proportion of the ends of the plurality of the cell-free DNAs located in the interior of nucleosomes is smaller than or equal to the first threshold, and/or, when the proportion of the ends of the plurality of the cell-free DNAs located onlinker DNAs is greater than or equal to the second threshold: or
determining that the sample is a non-cancer sample when a distance between the ends of the plurality of the cell-free DNAs and the center positions of the nucleosomes is greater than a third threshold: and determining that the sample is a cancer sample when the distance between the ends of the plurality of the cell-free DNAs and the center positions of the nucleosomes is smaller than or equal to the third threshold.

4. The classification method according to claim 2, wherein the position result is *N-index*, which meets

$$N - index = \frac{N_{\text{interior of nucleosome}}}{N_{\text{all}}}$$

wherein. $N_{\text{interior of nucleosome}}$ is the number of cell-free DNAs whose ends located in the interior of nucleosomes in the plurality of the cell-free DNAs: $N_{\text{all}}$ is the total number of the plurality of the cell-free DNAs;
or, the position result is *N'-index,* which meets

$$N' - index = \frac{N_{\text{interior of nucleosome}}}{N_{\text{linker DNA}}}$$

wherein. $N_{\text{linker DNA}}$ is the number of cell-free DNAs whose ends are located on linker DNAs between the nucleosomes:

or, the position result is *N''-index,* which meets

$$N'' - index = \sum_{i=1}^{n} a_i b_i$$

wherein, *n* is the number of the ends of the plurality of the cell-free DNAs, $a_i$ is a distance between an *i*-th end and the center of corresponding nucleosome, and $b_i$ is a weight of the *i*-th end:
preferably, determining the type of the sample according to the position result comprises:

determining that the sample is a cancer sample when *N-index* is greater than a first cutoff value: and

determining that the sample is a non-cancer sample when *N-index* is smaller than or equal to the first cutoff value:

preferably, determining the type of the sample according to the position result comprises:

determining that the sample is a non-cancer sample when *N'-index* is greater than a second cutoff value: and

determining that the sample is a cancer sample when *N'-index* is smaller than or equal to the second cutoff value:

preferably, determining the type of the sample according to the position result comprises:

determining that the sample is a non-cancer sample when *N''-index* is greater than a third cutoff value: and

determining that the sample is a cancer sample when *N''-index* is smaller than or equal to third cutoff value:

5. The classification method according to any one of claims 1 to 4. further comprising acquiring at least one selected from cell-free DNA copy number variation data, cell-free DNA methylation data, cell-free DNA length distribution data, cell-free DNA end base distribution data, cell-free DNA end distribution consistency data, marker data and image data of a sample for analysis, and then determining the classification of the sample in combination with the analysis result and the position result.

6. A computer-readable storage medium, storing computer-executable instructions which, when executed by a computer, cause the computer to perform the classification method according to any one of claims 1 to 5.

7. A device, comprising a processor and a memory, wherein the memory stores a computer program runnable on the processor which, when executed by the processor, causes the processor to perform the classification method according to any one of claims 1 to 5.

8. A system for diagnosing a cancer, comprising:

an acquisition module for acquiring sequencing data of cell-free DNA from a sample: and

a comparison module for aligning the sequencing data of the cell-free DNA with a position of a nucleosome on a reference genome to obtain the position result where the end of the cell-free DNA is relative to the nucleosome distribution, and judging whether the sample is a cancer sample according to the position result.

9. A Pan-cancer blood diagnosis marker, wherein the Pan-cancer blood diagnosis marker comprises at least one selected from *N-index, N'-index, N''-index,* which characterize the consistency between the fragmentation pattern of cell-free DNA and the nucleosome distribution of blood cells, which meets

$$N - index = \frac{N_{\text{interior of nucleosome}}}{N_{\text{all}}}$$

wherein, $N_{\text{interior of nucleosome}}$ is the number of cell-free DNAs whose ends located in the interior of nucleosomes in the plurality of the cell-free DNAs: and $N_{\text{all}}$ is the total number of the plurality of the cell-free DNAs;

$$N' - index = \frac{N_{\text{interior of nucleosome}}}{N_{\text{linker DNA}}}$$

wherein, $N_{\text{linker DNA}}$ is the number of cell-free DNAs whose ends are located on linker DNAs between the nucleosomes;

$$N'' - index = \sum_{i=1}^{n} a_i b_i$$

wherein, $n$ is the number of the ends of the plurality of the cell-free DNAs, $a_i$ is a distance between an i-th end and the center of corresponding nucleosome, and $b_i$ is a weight of the $i$-th end.

10. Use of the Pan-cancer blood diagnosis marker according to claim 9, or a product thereof for detecting the Pan-cancer blood diagnosis marker in the preparation of a cancer diagnosis product.

Acquire sequencing data of cell-free DNA of a sample S110

Align the sequencing data of cell-free DNA with a position of a nucleosome on a reference genome to obtain a position result of ends of cell-free DNA relative to nucleosome distribution S120

Determine the type of the sample according to the position result S130

FIG. 1

FIG. 2

FIG. 3

FIG. 4

FIG. 5

FIG. 6

FIG. 7

FIG. 8

FIG. 9

## INTERNATIONAL SEARCH REPORT

| International application No. |
| --- |
| **PCT/CN2023/093013** |

| A. | CLASSIFICATION OF SUBJECT MATTER |
| --- | --- |

G16H50/20(2018.01)i; G16B20/50(2019.01)i; G16B20/30(2019.01)i; C12Q1/68(2018.01)i

According to International Patent Classification (IPC) or to both national classification and IPC

| B. | FIELDS SEARCHED |
| --- | --- |

Minimum documentation searched (classification system followed by classification symbols)

IPC:G16H G16B C12Q

Documentation searched other than minimum documentation to the extent that such documents are included in the fields searched

Electronic data base consulted during the international search (name of data base and, where practicable, search terms used)

CNTXT; ENTXT; ENTXTC; DWPI; CNKI; ISI WEB OF SCIENCES; PUBMED: 游离DNA, 末端, 测序, 核小体, 位置, 癌, 肿瘤, cfDNA, terminal, sequencing, nucleosome, location, cancer, tumor

| C. | DOCUMENTS CONSIDERED TO BE RELEVANT | |
| --- | --- | --- |

| Category* | Citation of document, with indication, where appropriate, of the relevant passages | Relevant to claim No. |
| --- | --- | --- |
| Y | CN 115019952 A (SHENZHEN BAY LABORATORY) 06 September 2022 (2022-09-06) description, paragraphs 4-52 and 70-108 | 1-10 |
| Y | WO 2021130356 A1 (VIB VZW et al.) 01 July 2021 (2021-07-01) description, pages 10 and 15-16 | 1-10 |
| Y | WO 2022046947 A1 (GUARDANT HEALTH, INC.) 03 March 2022 (2022-03-03) description, paragraphs 97-104 | 1-10 |
| Y | HUANG, Qiangqing et al. "Bioinformatics Analysis for Circulating Cell-Free DNA in Cancer" *Cancers*, 11 June 2019 (2019-06-11), abstract, and page 8, paragraphs 3-5 | 1-10 |
| A | CN 114999567 A (THE CHINESE UNIVERSITY OF HONG KONG) 02 September 2022 (2022-09-02) claims 1-10 | 1-10 |
| A | CN 112805563 A (THE JOHNS HOPKINS UNIVERSITY) 14 May 2021 (2021-05-14) claims 1-67 | 1-10 |

☑ Further documents are listed in the continuation of Box C.  ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| **11 August 2023** | **22 August 2023** |

| Name and mailing address of the ISA/CN | Authorized officer |
| --- | --- |
| **China National Intellectual Property Administration (ISA/CN)** **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2023/093013** |

### C. DOCUMENTS CONSIDERED TO BE RELEVANT

| Category* | Citation of document, with indication, where appropriate, of the relevant passages | Relevant to claim No. |
| --- | --- | --- |
| A | CN 113362897 A (BERRY ONCOLOGY CO., LTD.) 07 September 2021 (2021-09-07) claims 1-10 | 1-10 |
| A | LIU, Yaping. "At the Dawn: Cell-free DNA Fragmentomics and Gene Regulation" *British Journal of Cancer*, 23 November 2021 (2021-11-23), pages 379-390 | 1-10 |

Form PCT/ISA/210 (second sheet) (July 2022)

**INTERNATIONAL SEARCH REPORT**
Information on patent family members

International application No.

**PCT/CN2023/093013**

| Patent document cited in search report | | | Publication date (day/month/year) | Patent family member(s) | | | Publication date (day/month/year) |
|---|---|---|---|---|---|---|---|
| CN | 115019952 | A | 06 September 2022 | None | | | |
| WO | 2021130356 | A1 | 01 July 2021 | EP | 4081655 | A1 | 02 November 2022 |
| | | | | US | 2023042332 | A1 | 09 February 2023 |
| WO | 2022046947 | A1 | 03 March 2022 | EP | 4205126 | A1 | 05 July 2023 |
| CN | 114999567 | A | 02 September 2022 | HUE | 057821 | T2 | 28 June 2022 |
| | | | | IL | 257055 | A | 29 March 2018 |
| | | | | IL | 257055 | B | 01 January 2022 |
| | | | | EP | 3967775 | A1 | 16 March 2022 |
| | | | | US | 2023197201 | A1 | 22 June 2023 |
| | | | | ES | 2907697 | T3 | 26 April 2022 |
| | | | | EP | 3325664 | A1 | 30 May 2018 |
| | | | | EP | 3325664 | A4 | 02 January 2019 |
| | | | | EP | 3325664 | B1 | 29 December 2021 |
| | | | | US | 2020005895 | A1 | 02 January 2020 |
| | | | | US | 11605445 | B2 | 14 March 2023 |
| | | | | KR | 20180031742 | A | 28 March 2018 |
| | | | | JP | 2018524991 | A | 06 September 2018 |
| | | | | JP | 6931236 | B2 | 01 September 2021 |
| | | | | WO | 2017012592 | A1 | 26 January 2017 |
| | | | | AU | 2016295616 | A1 | 15 February 2018 |
| | | | | AU | 2016295616 | B2 | 02 June 2022 |
| | | | | SG | 10202106935 | UA | 30 August 2021 |
| | | | | JP | 2021184732 | A | 09 December 2021 |
| | | | | JP | 7284522 | B2 | 31 May 2023 |
| | | | | TW | 201718872 | A | 01 June 2017 |
| | | | | TWI | 730973 | B | 21 June 2021 |
| | | | | US | 2020005897 | A1 | 02 January 2020 |
| | | | | US | 11615865 | B2 | 28 March 2023 |
| | | | | TW | 202142697 | A | 16 November 2021 |
| | | | | HK | 1251264 | A1 | 25 January 2019 |
| | | | | AU | 2022224861 | A1 | 20 October 2022 |
| | | | | HK | 1247645 | A1 | 28 September 2018 |
| | | | | US | 2017024513 | A1 | 26 January 2017 |
| | | | | US | 10453556 | B2 | 22 October 2019 |
| | | | | CA | 2993362 | A1 | 26 January 2017 |
| | | | | PT | 3325664 | T | 03 March 2022 |
| | | | | DK | 3325664 | T3 | 07 March 2022 |
| | | | | IL | 288622 | A | 01 February 2022 |
| | | | | US | 2020005896 | A1 | 02 January 2020 |
| | | | | US | 11581063 | B2 | 14 February 2023 |
| CN | 112805563 | A | 14 May 2021 | JP | 2021525069 | A | 24 September 2021 |
| | | | | AU | 2019269679 | A1 | 17 December 2020 |
| | | | | WO | 2019222657 | A1 | 21 November 2019 |
| | | | | BR | 112020023587 | A2 | 09 February 2021 |
| | | | | US | 2022325343 | A1 | 13 October 2022 |
| | | | | US | 2020149118 | A1 | 14 May 2020 |
| | | | | US | 10975431 | B2 | 13 April 2021 |
| | | | | US | 2021198747 | A1 | 01 July 2021 |
| | | | | US | 2021254152 | A1 | 19 August 2021 |
| | | | | CA | 3100345 | A1 | 21 November 2019 |

Form PCT/ISA/210 (patent family annex) (July 2022)

**INTERNATIONAL SEARCH REPORT**
**Information on patent family members**

International application No.

**PCT/CN2023/093013**

| Patent document cited in search report | | | Publication date (day/month/year) | Patent family member(s) | | | Publication date (day/month/year) |
|---|---|---|---|---|---|---|---|
| | | | | EP | 3794348 | A1 | 24 March 2021 |
| | | | | EP | 3794348 | A4 | 09 March 2022 |
| | | | | US | 2020131571 | A1 | 30 April 2020 |
| | | | | US | 10982279 | B2 | 20 April 2021 |
| | | | | KR | 20210045953 | A | 27 April 2021 |
| CN | 113362897 | A | 07 September 2021 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210496595

**Non-patent literature cited in the description**

- **SNYDER et al.** *Cell*, 14 January 2016, vol. 164 (1-2), 57-68
- **LIANG et al.** Whole-genome sequencing of cell-free DNA yields genome-wide read distribution patterns to track tissue of origin in cancer patients. *Clin Transl Med.*, October 2020, vol. 10 (6), e177
- **SONG et al.** 5-Hydroxymethylcytosine signatures in cell-free DNA provide information about tumor types and stages. *Cell Res.*, October 2017, vol. 27 (10), 1231-1242
- **CRISTIANO et al.** Genome-wide cell-free DNA fragmentation in patients with cancer. *Nature*, 2019, vol. 570, 385-389
- **ZHANG et al.** Hypomethylation in HBV integration regions aids non-invasive surveillance to hepatocellular carcinoma by low-pass genome-wide bisulfite sequencing. *BMC Medicine*, 03 August 2020, vol. 18 (1), 200
- **CHAN et al.** *PNAS*, 19 November 2013, vol. 110 (47), 18761-8